# EUROPÄISCHE PATENTSCHRIFT

(11) **EP 3 348 201 B1**
(45) Veröffentlichungstag und Bekanntmachung des Hinweises auf die Patenterteilung: **10.07.2019**
(21) Anmeldenummer: 17190163.0
(22) Anmeldetag: 08.09.2017
(51) Int. Cl.: A61B 6/04, G06F 3/01

(54) **VERFAHREN ZUM POSITIONIEREN EINER PATIENTENLIEGE UND PATIENTENLIEGE**
METHOD FOR POSITIONING A PATIENT SUPPORT AND PATIENT SUPPORT
PROCÉDÉ DE POSITIONNEMENT D'UN LIT D'EXAMEN ET LIT D'EXAMEN

(43) Veröffentlichungstag der Anmeldung: 18.07.2018
(73) Patentinhaber: Siemens Healthcare GmbH, 91052 Erlangen (DE)
(72) Erfinder: Holzki, Norbert, 91058 Erlangen (DE)

(56) Entgegenhaltungen:
- DE-A1-102006 051 881
- DE-U1-202016 007 430
- JP-A- H01 305 934
- JP-A- H11 137 543
- US-A1- 2015 277 420

## Beschreibung

Die Erfindung betrifft ein Verfahren zum Positionieren einer Patientenliege, insbesondere einer bildgebenden medizinischen Einrichtung, umfassend eine Benutzerschnittstelle zum Erfassen einer Bedieneingabe.

Bildgebende medizinische Einrichtung, wie beispielsweise Magnetresonanztomographen oder bildgebende Röntgeneinrichtungen, insbesondere Computertomographen, die dazu ausgebildet sind, das Körperinnere eines Patienten zu erfassen, sind aus dem Stand der Technik hinlänglich bekannt. Für gewöhnlich befindet sich der Patient zur Untersuchung auf einer Patientenliege (auch: Patiententisch), die insbesondere in horizontaler und/oder vertikaler Richtung geeignet positioniert werden muss, damit der untersuchungsrelevante Bereich erfasst werden kann. Die Patientenliege wird bei gängigen Ausführungen über Schaltelemente wie Knöpfe, Fußschalter oder dergleichen bedient.

Insbesondere bei Computertomographen erfolgt die Bedienung über Bedienpanels an der Gantry. Die Steuerung der translatorischen Bewegungsfreiheitsgrade erfolgt relativ grob über Drucktasten, die gegebenenfalls intervallweise betätigt werden. Bei gängigen Ausführungen des Standes der Technik ist für jede Bewegungsrichtung (hoch/runter, rein/raus) ein eigener Schalter bzw. eine eigene Drucktaste vorgesehen. Die Verfahrgeschwindigkeit der Patientenliege ist typischerweise fest vorgegeben, gegebenenfalls kann vorgesehen sein, die Verfahrgeschwindigkeit über eine weitere Taste zwischen einem Normalbetrieb und einem Betrieb mit erhöhter Geschwindigkeit umzuschalten. Die genaue Positionierung des Patienten bzw. der Patientenliege ist damit relativ umständlich und ineffizient.

Aus den Dokumenten DE-A1-10 2006 051881; JP-A-H11 137543; US-A1-2015/277420; JP-A-H01 305934 und DE-20 2016 007430 U1 sind verschiedene Benutzerschnittstelle mit einem Bedienelement zum Erfassen einer Bedieneingabe bekannt, bei denen zumindest ein Bewegungsfreiheitsgrad der Patientenliege in Abhängigkeit einer vom Benutzer auf das Bedienelement ausgeübten Kraft verstellt wird.

Die Erfindung stellt sich zur Aufgabe, eine verbessertes Verfahren zum Positionieren einer Patientenliege anzugeben, welches insbesondere ein intuitives und effizientes Anfahren an eine Zielposition ermöglicht.

Hinsichtlich des Verfahrens wird die vorstehend genannte Aufgabe gelöst durch Merkmale des Patentanspruchs 1.

Hinsichtlich der Vorrichtung wird die vorstehend genannte Aufgabe gelöst durch Merkmale des Patentanspruchs 7 bzw. 8. Vorteilhafte Ausgestaltungen der Erfindung sind Gegenstand der Unteransprüche.

Bei einem Verfahren zum Betrieb einer Patientenliege, insbesondere einer bildgebenden medizinischen Einrichtung, ist eine Benutzerschnittstelle mit einem Bedienelement zum Erfassen einer Bedieneingabe vorgesehen. Gemäß der Erfindung wird zumindest ein Bewegungsfreiheitsgrad der Patientenliege in Abhängigkeit einer vom Benutzer auf das Bedienelement ausgeübten Kraft verstellt.

Grundlegender Gedanke der Erfindung ist es, die vom Benutzer auf das Bedienelement ausgeübte Kraft zu erfassen und die Patientenliege insbesondere hinsichtlich translatorischer Bewegungsfreiheitsgrade nach Maßgabe der erfassten Kraft zu steuern. Die erfasste Kraft beinhaltet insbesondere den Betrag der vom Benutzer ausgeübten Kraft.

Der Bewegungsfreiheitsgrad der Patientenliege (auch: Patiententisch) im Sinne dieser Spezifikation betrifft ein Verstellen der Patientenliege hinsichtlich ihrer räumlichen Konfiguration. Insbesondere betrifft der Bewegungsfreiheitsgrad eine translatorische Bewegung der Patientenliege in horizontaler oder vertikaler Richtung oder eine Dreh- bzw. Schwenkbewegung, beispielsweise das Verstellen eines Neigungswinkels einer Kopf- oder Fußstütze.

Das Bedienelement ist zum Erfassen der darauf ausgeübten Kraft, insbesondere des darauf ausgeübten Druckes ausgebildet. Das Bedienelement ist hierzu in einer möglichen Ausgestaltung als kraftabhängiger Schalter oder Drucktaste mit entsprechender Mechanik und elektrischer Schnittstelle ausgeführt. Bevorzugt erfolgt die Erfassung der Bedieneingabe zumindest zum Teil kapazitiv, insbesondere projiziertkapazitiv. Das Bedienelement ist als Tastensymbol, insbesondere Widget, eines Displays (auch: Force-Touch-Display) mit berührungssensitiver Oberfläche realisiert. Zum Erfassen der vom Benutzer ausgeübten Kraft sind beispielsweise separate Drucksensoren vorgesehen. In einer anderen Ausgestaltung wird die vom Benutzer ausgeübte Kraft anhand einer gemessenen Durchbiegung einer das Display abdeckenden Abdeckscheibe erfasst. Hierzu kann insbesondere vorgesehen sein, die Abdeckscheibe am Display mittels optischen Bondings zu befestigen. Alternativ oder zusätzlich wird die vom Benutzer auf das Bedienelement ausgeübte Kraft anhand der Ausdehnung eines Berührungspunktes bzw. einer Berührungsfläche auf dem Display erfasst. Bevorzugt erfolgt eine redundante Signalauswertung der zum Erfassen der Bedieneingabe und der zum Erfassen der Kraft bzw. des Betrags der ausgeübten Kraft vorgesehenen Sensoren bzw. Messeinrichtungen derart, dass eine mit der Bedieneingabe verknüpfte Funktionalität, die im vorliegenden Fall das Verstellen des Bewegungsfreiheitsgrades der Patientenliege betrifft, erst dann erfolgt, wenn entsprechend konsistente Signale vorliegen. Es ist insbesondere vorgesehen, dass die von der Benutzerschnittstelle übertragenen Signale zum Positionieren der Patientenliege von der Benutzerschnittstelle doppelkanalig übertragen werden. Dabei dient ein Kanal beispielsweise zur Übertragung eines nach Maßgabe der erfassten Kraft generierten Signals und ein weiterer Kanal beispielsweise zur Übermittlung von Koordinaten.

Bevorzugt wird eine Verstellgeschwindigkeit in Abhängigkeit der auf das Bedienelement ausgeübten Kraft angepasst. Ein Benutzer wird in der Regel mit einem höheren Kraftaufwand auf das Bedienelement einwirken, wenn er die Patientenliege mit hoher Verstellgeschwindigkeit positionieren bzw. verfahren will. Bei Annäherung an die Zielposition wird der Druck erniedrigt, um eine Verlangsamung der Bewegung zu bewirken. Auf diese Art und Weise ist eine sehr intuitive Bedienung und ein effizientes Anfahren an die Zielposition ermöglicht.

In einer möglichen Ausgestaltung des erfindungsgemäßen Verfahrens wird die Verstellgeschwindigkeit derart angepasst, dass diese direkt proportional zur ausgeübten Kraft ist. Die Kraft-Verstellgeschwindigkeit-Kennlinie ist in diesem Fall eine Gerade. In anderen Fällen kann die Kraft-Verstellgeschwindigkeit-Kennlinie anderweitig vorgegeben werden. Beispielsweise kann die Kraft-Verstellgeschwindigkeit-Kennlinie einen Verlauf aufweisen, der abschnittsweise von Geraden mit unterschiedlichen Steigungen gebildet ist.

Bevorzugt wird die Verstellgeschwindigkeit in Abhängigkeit einer Abbildungsfunktion gewählt, die Kräften aus einem Kraftintervall entsprechend Verstellgeschwindigkeiten aus einem Geschwindigkeitsintervall zuordnet. Die Abbildungsfunktion kann beispielsweise als Software in einer Auswertelektronik der Benutzerschnittstelle implementiert sein, die insbesondere zumindest einen Prozessor, integrierten Schaltkreis, Mikroprozessor, Mikrocontroller und/oder Mikrochip umfasst. Die Abbildungsfunktion ist insbesondere eine Implementierung der bereits genannten Kraft-Verstellgeschwindigkeit-Kennlinie für Kräfte, die innerhalb des Kraftintervalls liegen. Wird auf das Bedienelement eine Kraft ausgeübt, die außerhalb des definierten Kraftintervalls liegt, so wird dies typischerweise nicht mit einer Gerätefunktion, insbesondere nicht mit dem Verstellen eines Bewegungsfreiheitsgrades der Patientenliege verknüpft.

Die Abbildungsfunktion ist vorzugsweise monoton, insbesondere streng monoton steigend. Bevorzugt ist die Korrelation zwischen Verstellgeschwindigkeit und ausgeübter Kraft derart, dass ein höherer Druck bzw. eine höhere Kraftausübung zu schnelleren Bewegungen bzw. höheren Verstellgeschwindigkeiten korrespondiert. Vorzugsweise sind verschiedene Abbildungsfunktionen für unterschiedliche Bewegungsfreiheitsgrade der Patientenliege implementiert. Der Verlauf der Abbildungsfunktion kann insbesondere vom Anwendungsfall bzw. vom Workflow abhängig sein.

Zur Implementierung von sanften Bewegungsprofilen ist bevorzugt vorgesehen, der Abbildungsfunktion entsprechend geglättete Kraft-Verstellgeschwindigkeit-Kennlinien zu Grunde zulegen.

Vorzugsweise wird eine Rückmeldung optisch, haptisch und/oder akustisch ausgegeben, wenn die auf das Bedienelement ausgeübte Kraft kleiner als ein vorgegebener oder vorgebbarer Minimalwert ist. Die Rückmeldung kann beispielsweise dem Benutzer Betriebsbereitschaft signalisieren. Visuelles bzw. optisches Feedback erfolgt beispielsweise über das Display. Hierzu kann insbesondere die Farbgestaltung eines Tastensymbols verändert werden. Eine haptische Rückmeldung kann beispielsweise durch kurzes Vibrieren, beispielsweise der berührungssensitiven Oberfläche, erfolgen. Entsprechend erfolgt die Ausgabe einer akustischen Rückmeldung typischerweise über Lautsprecher.

Es werden hochfrequente Anteile der auf das Bedienelement ausgeübten Kraft herausgefiltert. Dies kann insbesondere anhand einer entsprechenden Tiefpassfilterung des Signals erfolgen, das in Abhängigkeit der vom Benutzer ausgeübten Kraft generiert wurde.

Die vorstehend genannte Patientenliege umfasst die Benutzerschnittstelle mit dem zumindest einem Bedienelement zum Erfassen der Bedieneingabe und eine Steuereinheit zum Steuern des zumindest einen Bewegungsfreiheitsgrades der Patientenliege. Gemäß der Erfindung steht die Benutzerschnittstelle derart mit der Steuereinheit in einer Wirkverbindung, dass der Bewegungsfreiheitsgrad der Patientenliege gemäß dem bereits beschriebenen Verfahren verstellbar, insbesondere motorisch verstellbar ist. Die damit verknüpften Vorteile ergeben sich unmittelbar aus der vorangehenden Beschreibung mit Bezug auf das Verfahren zum Positionieren der Patientenliege.

Die Patientenliege ist besonders bevorzugt Teil einer bildgebenden medizinischen Einrichtung, insbesondere eines Computertomographen. Die durch die Erfindung bewirkte Effizienzsteigerung bzw. Zeitersparnis ist insbesondere bei derartigen bildgebenden medizinischen Einrichtungen, die oftmals eine hohe Auslastung aufweisen, von großer Bedeutung. Da zudem ermöglicht ist, besonders sanfte Bewegungsprofile zu implementieren, kann sich im Allgemeinen eine positive Patientenerfahrung einstellen.

Softwareimplementierungen, insbesondere in Elektronikkomponenten wie etwa der vorstehend genannten Auswerteelektronik, sind bevorzugt in einem nichtflüchtigem bzw. persistentem Speichermedium realisiert.

Die oben beschriebenen Eigenschaften, Merkmale und Vorteile der Erfindung sowie die Art und Weise, wie diese erreicht werden, werden klarer und deutlicher verständlich im Zusammenhang mit der folgenden Beschreibung der Ausführungsbeispiele, die mit Bezug auf die Zeichnungen näher erläutert werden.

Für eine weitere Beschreibung der Erfindung wird auf die in den Zeichnungsfiguren gezeigten Ausführungsbeispiele verwiesen. Es zeigen in einer schematischen Darstellung:

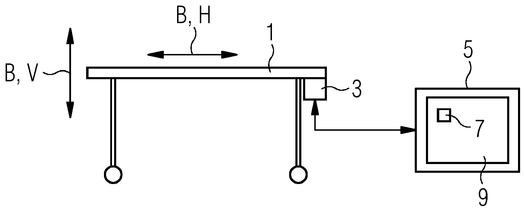

- Fig. 1:: den schematischen Ablauf eines Verfahrens zur Positionierung einer Patientenliege.

Figur 1 illustriert schematischen den Ablauf eines Verfahrens zum Positionieren einer Patientenliege 1. Die exemplarisch dargestellte Patientenliege 1 ist hinsichtlich mehrerer translatorischer Bewegungsfreiheitsgrade B motorisch verstellbar, wobei eine vertikale Höhenverstellung V und eine Horizontalbewegung H in Figur 1 schematisch durch Doppelpfeile angedeutet sind.

Die Patientenliege 1 kann insbesondere eine Komponente einer nicht näher dargestellten bildgebenden medizinischen Einrichtung sein.

Das Verstellen der Bewegungsfreiheitsgrade B erfolgt mittels einer Steuereinheit 3, die in nicht näher dargestellter Art und Weise Stellglieder, insbesondere Elektromotoren ansteuert. Eine Benutzerschnittstelle 5 steht hierzu mit der Steuereinheit 3 in einer Wirkverbindung und sendet Signale an die Steuereinheit 3, welche von einem auf ein Bedienelement 7 der Benutzerschnittstelle 5 ausgeübten Druck abhängig sind.

Die Benutzerschnittstelle 5 umfasst ein Display 9 mit berührungssensitiver Oberfläche. Das Bedienelement 7 ist in dem lediglich exemplarisch dargestellten Ausführungsbeispiel ein Tastensymbol, das auf dem Display 9 angezeigt wird und dient zur Erfassung einer Benutzereingabe, welche das Verstellen eines Bewegungsfreiheitsgrades B betrifft.

Das Display 9 ist dazu ausgebildet, die vom Benutzer auf das Bedienelement 7 ausgeübte Kraft F zu erfassen. Das so erfasste Signal wird gegebenenfalls nach einer geeigneten Tiefpassfilterung in Relation zu einer Verstellgeschwindigkeit gesetzt. Hierzu ist in einer Elektronikkomponente der Benutzerschnittstelle 5 eine entsprechende Software, insbesondere in Form einer Abbildungsfunktion implementiert, welche der erfassten Kraft F eine Verstellgeschwindigkeit zuordnet.

Bei Betätigung des Bedienelements 7 erfolgt ein Verstellen des damit verknüpften Bewegungsfreiheitsgrades B nur, wenn die auf das Bedienelement 7 ausgeübte Kraft F innerhalb eines vorgegebenen Kraftintervalls liegt. Die Abbildungsfunktion ist vorzugsweise als streng monoton steigende Funktion implementiert, die das Kraftintervall [Fₘᵢₙ, Fₘₐₓ] auf ein entsprechendes Geschwindigkeitsintervall [Vₘᵢₙ, Vₘₐₓ] abbildet. Somit wird die Verstellgeschwindigkeit, mit der der Bewegungsfreiheitsgrad B angepasst wird, erhöht, wenn auf das Bedienelement 7 eine größere Kraft F ausgeübt wird.

Übt der Benutzer lediglich eine Kraft F auf, die kleiner als ein vorgegebener Minimalwert Fₘᵢₙ ist, so wird ein visuelles Feedback bzw. eine visuelle Rückmeldung ausgeben. Dabei verändert das Tastensymbol seine Farbe. Alternativ oder zusätzlich kann ein akustisches oder haptisches Feedback ausgegeben werden.

Entspricht die ausgeübte Kraft F dem Minimalwert Fₘᵢₙ, so wird der mit dem entsprechenden Bedienelement 7 verknüpfte Bewegungsfreiheitsgrad B mit der minimalen Verstellgeschwindigkeit Vₘᵢₙ verstellt. Entspricht die ausgeübte Kraft F dem Maximalwert Fₘₐₓ, so wird mit der maximalen Verstellgeschwindigkeit Vₘₐₓ gefahren. Der Verlauf der Abbildungsfunktion variiert im Allgemeinen für jeden Bewegungsfreiheitsgrad B und kann zusätzlich vom Betriebskontext, Anwendungsfall bzw. Workflow abhängen.

Obwohl die Erfindung im Detail mit Bezug auf die bevorzugten Ausführungsbeispiele näher illustriert und beschrieben wurde, so ist die Erfindung nicht hierdurch eingeschränkt. Andere Variationen und Kombinationen können vom Fachmann hieraus abgeleitet werden, ohne vom wesentlichen Gedanken der Erfindung zu abzuweichen.

## Patentansprüche

1. Verfahren zum Betrieb einer Patientenliege (1), insbesondere einer bildgebenden medizinischen Einrichtung, umfassend eine Benutzerschnittstelle (5) mit einem Bedienelement (7) zum Erfassen einer Bedieneingabe, wobei zumindest ein Bewegungsfreiheitsgrad (B) der Patientenliege (1) in Abhängigkeit einer vom Benutzer auf das Bedienelement (7) ausgeübten Kraft verstellt wird, **dadurch gekennzeichnet, dass** das Bedienelement (7) als Tastensymbol eines Displays (9) mit berührungssensitiver Oberfläche realisiert ist und hochfrequente Anteile der auf das Bedienelement (7) ausgeübten Kraft herausgefiltert werden.

2. Verfahren nach Anspruch 1, **dadurch gekennzeichnet, dass** eine Verstellgeschwindigkeit in Abhängigkeit der auf das Bedienelement (7) ausgeübten Kraft angepasst wird.

3. Verfahren nach Anspruch 2, **dadurch gekennzeichnet, dass** die Verstellgeschwindigkeit derart angepasst wird, dass diese direkt proportional zur ausgeübten Kraft ist.

4. Verfahren nach Anspruch 2 oder 3, **dadurch gekennzeichnet, dass** eine Verstellgeschwindigkeit in Abhängigkeit einer Abbildungsfunktion gewählt wird, die Kräften aus einem Kraftintervall entsprechend Verstellgeschwindigkeiten aus einem Geschwindigkeitsintervall zuordnet.

5. Verfahren nach Anspruch 4, **dadurch gekennzeichnet, dass** die Abbildungsfunktion monoton, insbesondere streng monoton steigend ist.

6. Verfahren nach einem der vorherigen Ansprüche, **dadurch gekennzeichnet, dass** eine Rückmeldung optisch, haptisch und/oder akustisch ausgegeben wird, wenn die auf das Bedienelement (7) ausgeübten Kraft kleiner als ein vorgegebener oder vorgebbarer Minimalwert ist.

7. Patientenliege (1), umfassend eine Benutzerschnittstelle (5) mit zumindest einem Bedienelement (7) zum Erfassen einer Bedieneingabe, wobei das Bedienelement als Tastensymbol eines Displays (9) mit berührungssensitiver Oberfläche realisiert ist und hochfrequente Anteile der auf das Bedienelement ausgeübten Kraft herausgefiltert werden, und einer Steuereinheit (3) zum Steuern zumindest eines Bewegungsfreiheitsgrades (B) der Patientenliege (1), wobei die Benutzerschnittstelle (5) derart mit der Steuereinheit (3) in einer Wirkverbindung steht, dass der Bewegungsfreiheitsgrad (B) der Patientenliege (1) gemäß einem Verfahren nach einem der vorhergehenden Ansprüche 1 bis 6 verstellbar ist.

8. Bildgebenden medizinischen Einrichtung, insbesondere Computertomograph, mit einer Patientenliege (1) nach Anspruch 7.

## Claims

1. Method for operating a patient couch (1), in particular a medical imaging device, comprising a user interface (5) with a control element (7) for acquiring a control input, wherein at least one degree of motion (B) of the patient couch (1) is adjusted as a function of a force exerted by the user on the control element (7), **characterised in that** the control element (7) is realised as a button symbol of a display (9) with a touch-sensitive surface and high-frequency components of the force exerted onto the control element (7) are filtered out.

2. Method according to claim 1, **characterised in that** an adjusting speed is adapted as a function of the force exerted onto the control element (7).

3. Method according to claim 2, **characterised in that** the adjusting speed is adapted such that this is directly proportional to the exerted force.

4. Method according to claim 2 or 3, **characterised in that** an adjusting speed is selected as a function of a mapping function, which correspondingly assigns adjustment speeds from a speed interval to forces from a force interval.

5. Method according to claim 4, **characterised in that** the mapping function is monotonically increasing, in particular strictly monotonically increasing.

6. Method according to one of the preceding claims, **characterised in that** a feedback is output visually, haptically and/or acoustically, if the force exerted onto the control element (7) is less than a predetermined or predeterminable minimum value.

7. Patient couch (1) comprising a user interface (5) with at least one control element (7) for acquiring a control input, wherein the control element is realised as a button symbol of a display (9) with a touch-sensitive surface and high-frequency components of the force exerted onto the control element are filtered out, and a control unit (3) for controlling at least one degree of motion (B) of the patient couch (1), wherein the user interface (5) is actively connected to the control unit (3) such that the degree of motion (B) of the patient couch (1) can be adjusted in accordance with a method according to one of the preceding claims 1 to 6.

8. Medical imaging device, in particular computed tomograph having a patient couch (1) according to claim 7.

## Revendications

1. Procédé pour faire fonctionner une couchette (1) de patient, notamment d'un dispositif médical d'imagerie, comprenant une interface (5) d'utilisateur ayant un élément (7) de commande pour détecter une entrée de commande, au moins un degré (B) de liberté de déplacement de la couchette (1) du patient étant réglé en fonction d'une force appliquée par l'utilisateur sur l'élément (7) de commande, **caractérisé en ce que** l'élément (7) de commande est réalisé sous la forme d'un symbole de touche d'un affichage (9) à surface sensible au toucher et des parties de haute fréquence de la force appliquée à l'élément (7) de commande sont éliminées par filtrage.

2. Procédé suivant la revendication 1, **caractérisé en ce que** l'on adapte une vitesse de déplacement en fonction de la force appliquée à l'élément (7) de commande.

3. Procédé suivant la revendication 2, **caractérisé en ce que** l'on adapte la vitesse de déplacement de manière à ce qu'elle soit directement proportionnelle à la force appliquée.

4. Procédé suivant la revendication 2 ou 3, **caractérisé en ce que** l'on choisit une vitesse de déplacement en fonction d'une fonction de reproduction, qui est associée à des forces dans un intervalle de force correspondant à une vitesse de déplacement dans un intervalle de déplacement.

5. Procédé suivant la revendication 4, **caractérisé en ce que** la fonction de reproduction est monotone, notamment croissante d'une manière strictement monotone.

6. Procédé suivant l'une des revendications précédentes, **caractérisé en ce que** l'on émet un message de retour visuellement, haptiquement et/ou acoustiquement, si la force appliquée à l'élément (7) de commande est plus petite qu'une valeur minimum donnée à l'avance ou pouvant l'être.

7. Couchette (1) de patient comprenant une interface (5) d'utilisateur ayant au moins un élément (7) de commande pour détecter une entrée de commande, l'élément de commande étant réalisé sous la forme d'un symbole de touche d'un affichage (9) à surface sensible au toucher et des parties de haute fréquence de la force appliquée à l'élément (7) de commande sont éliminées par filtrage, et une unité (3) de commande pour commander au moins un degré (B) de liberté de déplacement de la couchette (1) du patient, l'interface (5) d'utilisateur étant en liaison d'action avec l'unité (3) de commande de manière à pouvoir régler le degré (B) de liberté de déplacement de la couchette (1) du patient selon un procédé suivant l'une des revendications 1 à 6 précédentes.

8. Dispositif médical d'imagerie, notamment tomographe à ordinateur, ayant une couchette (1) de patient suivant la revendication 7.
